# EUROPEAN PATENT SPECIFICATION

(11) **EP 3 079 119 B1**
(45) Date of publication and mention of the grant of the patent: **01.04.2020**
(21) Application number: 14867996.2
(22) Date of filing: 21.11.2014
(51) Int. Cl.: G06F 21/31, G06F 21/32, G06Q 50/22, A61B 5/021, A61B 5/11, A61B 5/053, A61B 5/024, A61B 5/0205

(54) **USER AUTHENTICATION SYSTEM**
BENUTZERAUTHENTIFIZIERUNGSSYSTEM
SYSTÈME D'AUTHENTIFICATION D'UTILISATEURS

(30) Priority: 04.12.2013 JP 2013251099
(43) Date of publication of application: 12.10.2016
(73) Proprietor: Omron Healthcare Co., Ltd., Kyoto 617-0002 (JP)
(72) Inventor: YAMASHITA, Shingo, Muko-shi Kyoto 617-0002 (JP); SAWANOI, Yukiya, Muko-shi Kyoto 617-0002 (JP); MORITA, Kazuyuki, Muko-shi Kyoto 617-0002 (JP)
(74) Representative: Isarpatent
(86) International application number: PCT/JP2014/080973
(87) International publication number: WO 2015/083568

(56) References cited:
- EP-A2- 1 362 430
- WO-A1-2013/096954
- JP-A- 2004 030 464
- JP-A- 2004 358 232
- JP-A- 2005 354 490
- JP-A- 2006 217 930
- US-A1- 2003 190 062

## Description

### Technical Field

The present invention relates to a user authentication system for, when new bodily information for a certain user is registered, performing authentication of whether or not the new bodily information relates to the user.

### Background Art

A process has been performed in which multiple types of bodily information acquisition apparatuses (e.g., blood pressure meters, activity meters, body composition meters, etc.) are used to sequentially acquire different types of bodily information (e.g., blood pressure, activity amount, body composition, etc.) for a user, the acquired bodily information is associated with user information specifying the user, and is then sequentially integrated into an information management system, and the integrated bodily information is called on as needed so as to be put to use for health management or medical diagnosis of the user. Unique apparatus identification numbers for specifying the bodily information acquisition apparatuses are allocated to the multiple bodily information acquisition apparatuses to be used, and the information of the apparatus identification numbers is added to the bodily information. Thus, regarding the integrated bodily information, there is extremely little risk that wrong information will be integrated with respect to information specifying the bodily information acquisition apparatus used to acquire the integrated bodily information.

However, sometimes a bodily information acquisition apparatus is used jointly by multiple users. For example, in a household, there are cases where a blood pressure meter is used jointly between married couples or between parents and children. Unique user identification numbers are allocated to the multiple users in order to specify each user. However, during use of a bodily information acquisition apparatus, a user identification number is sometimes input erroneously into a bodily information acquisition apparatus. That is, a case may occur in which bodily information for another user (e.g., a son) who is not a certain user (e.g., a father) is integrated into the information management system. Accordingly, regarding the integrated bodily information, there is a risk that wrong information will be integrated with respect to user information specifying the user to whom the bodily information belongs. In view of this, for example, Patent Literature 1 and 2 disclose that authentication of an individual is performed by comparing a new measurement value for bodily information with past measurement values. Patent Literature 3 relates to a wearable computing device for secure control of physiological sensors and medical devices, with secure storage of medical records and bioimpedence biometric. Patent Literature 4 relates to an apparatus for measuring biological data and exercise machines

### Citation List

### Patent Literature

Patent Literature 1: JP H11-76177A
Patent Literature 2: JP 2011-254954A
Patent Literature 3: WO 2013/096954 A1
Patent Literature 4: US 2003/190062 A1

### Summary of Invention

### Technical Problem

Patent Literature 1 discloses a health management assistance apparatus according to which an individual having past bodily information, such as body weight or body fat percentage, that falls within range of a predetermined numerical value centered about a new measurement value for the bodily information is recognized as a certain individual. Patent Literature 1 is problematic in that in the case where multiple people qualify as individuals having past bodily information that is similar to the new measurement value, it is difficult to specify which of the individuals is the actual individual, and therefore the accuracy of individual authentication is not good.

Patent Literature 2 discloses an exercise amount measurement system according to which bodily authentication by means of a finger print, a palm print, or the like is performed, and then based on the body weight of a user estimated based on measurement values for a load during a stepping exercise and a ratio between loads applied to the left and right feet of the user, the body weight of an authenticated person that has already been stored and the ratio between the left and right feet are compared so as to determine whether or not the user and the authenticated person are the same person. In Patent Literature 2, a finger print, a palm print, veins of a hand or finger, an iris, a retina, a voice print, a face shape, or the like is used as the source of information for body authentication, and not the load and exercise level, which are being measured. Also, Patent Literature 2 discloses that even if there is a match with authentication data of an individual registered by bodily authentication, the most recent recorded body weight data is compared to determine whether or not there is a discrepancy with the measured body weight, and when a difference in body weight of a normally-impossible degree is recognized, the measured body weight is recorded as referential data. However, the determination of whether or not there is a discrepancy in Patent Literature 2 is performed based on the new bodily information and the past bodily information acquired from the same bodily information acquisition apparatus, and the determination of whether or not there is a discrepancy is not determined by comparing information acquired using a certain bodily information acquisition apparatus and information acquired using another bodily information acquisition apparatus.

In view of this, the problem of the present invention lies in providing a user authentication system that can perform user recognition with higher accuracy based on acquired bodily information of a user in an environment in which multiple users use multiple types of bodily information acquisition apparatuses to acquire multiple different types of bodily information.

### Solution to Problem

In order to solve the foregoing problems, a user authentication system as defined in the appended claims is provided. Disclosed is also a user authentication system including:
a plurality of types of bodily information acquisition apparatuses configured to acquire different types of bodily information for a certain user;
a storage unit configured to store pieces of bodily information acquired by the respective bodily information acquisition apparatuses, in association with the certain user; and
a user determination unit configured to check whether or not there is a logical discrepancy between new bodily information acquired newly by a certain bodily information acquisition apparatus among the plurality of types of bodily information acquisition apparatuses and other bodily information acquired by a bodily information acquisition apparatus other than the certain bodily information acquisition apparatus, and thereby determine whether or not the new bodily information acquired by the certain bodily information acquisition apparatus relates to the certain user.

A "logical discrepancy" in the present specification means that acquisition of multiple pieces of new bodily information and position information is substantially impossible and cannot happen in reality from a temporal perspective.

"Different types of bodily information" in the present specification refers to blood pressure, activity amount, body weight, body composition, and the like.

With the user authentication system according to the present disclosure, it is checked whether or not there is a logical discrepancy between new bodily information acquired newly by a certain bodily information acquisition apparatus and other bodily information acquired by another bodily information acquisition apparatus, and if there is a logical discrepancy between the two, the user determination unit determines that the new bodily information does not relate to the certain user. Accordingly, user recognition can be performed with higher accuracy than in the case of performing user recognition by comparing new bodily information and past bodily information with a certain bodily information acquisition apparatus.

In another aspect, a user authentication system of the present disclosure includes:
one or more bodily information acquisition apparatuses configured to acquire different pieces of bodily information for a certain user;
a storage unit configured to store the bodily information acquired by the one or more bodily information acquisition apparatuses in association with the certain user;
a position information acquisition apparatus configured to acquire position information relating to the certain user; and
a user determination unit configured to check whether or not there is a logical discrepancy between new bodily information acquired newly by a certain bodily information acquisition apparatus among the one or more bodily information acquisition apparatuses and the position information acquired by the position information acquisition apparatus, and thereby determine whether or not the new bodily information acquired by the certain bodily information acquisition apparatus relates to the certain user.

With the user authentication system of the present disclosure, it is checked whether or not there is a logical discrepancy between the new bodily information and the position information, and it is thereby possible to perform user recognition with higher accuracy than in the case of performing user recognition by comparing only the new bodily information and past bodily information with the certain bodily information acquisition apparatus.

A user authentication system according to an aspect of the disclosure is such that a said storage unit is provided in each of the bodily information acquisition apparatuses and/or is provided independently of the bodily information acquisition apparatuses.

With the user authentication system, it is possible to obtain a flexible system configuration.

A user authentication system according to an aspect of the disclosure is such that if it is determined by the user determination unit that the new bodily information acquired by the certain bodily information acquisition apparatus does not relate to the certain user, attribute information indicating that the new bodily information does not relate to the certain user is attached to the new bodily information.

With the user authentication system, new bodily information that may not relate to the certain user can be easily distinguished from correct other bodily information.

The user authentication system may further include an information notification unit configured to perform notification of the new bodily information, information of the certain user, and information relating to the determination result, in which if it is determined by the user determination unit that the new bodily information acquired by the certain bodily information acquisition apparatus does not relate to the certain user, notification of a message relating to the determination result is performed by the information notification unit.

With the user authentication system, it is possible to warn a user that new bodily information may not relate to the certain user.

A user authentication system may further include an information notification unit configured to perform notification of the new bodily information, information of the certain user, and information relating to the determination result, in which if it is determined by the user determination unit that the new bodily information acquired by the certain bodily information acquisition apparatus does not relate to the certain user, notification of a message for confirming a handling policy of the certain user with respect to the determination result is performed by the information notification unit.

With the user authentication system, the will of the user can be checked with respect to a determination result indicating that the new bodily information does not relate to the certain user.

A user authentication system according to an aspect of the disclosure is such that if it is determined by the user determination unit that the new bodily information acquired by the certain bodily information acquisition apparatus does not relate to the certain user, the new bodily information is subjected to processing for storage in the storage unit and is not made available for other processing.

With the user authentication system according to this disclosure, the new bodily information is merely stored as backup information and thus it is not necessary to perform needless processing other than that.

### Advantageous Effects

As is clear from the above description, with the user authentication system of the present disclosure, if new bodily information acquired by a certain bodily information acquisition apparatus has a logical discrepancy in its relationship with other bodily information or position information, a user determination unit determines that the new bodily information does not relate to a certain user, and therefore user recognition can be performed with higher accuracy.

### Brief Description of Drawings

FIG. 1 is a functional block diagram showing a configuration of a user authentication system according to a disclosure of the present disclosure.
FIG. 2 is a functional block diagram for a blood pressure meter used in a user authentication system.
FIG. 3 is a functional block diagram for a body composition meter used in a user authentication system.
FIG. 4 is a functional block diagram for an activity meter used in a user authentication system.
FIG. 5 is a functional block diagram for a sleep meter used in a user authentication system.
FIG. 6 is a diagram showing a schematic operation flow for a user authentication system.
FIG. 7 is a diagram showing an operation flow of a user authentication system according to a first embodiment.
FIG. 8 is an example of display in the first embodiment.
FIG. 9 is a diagram showing an operation flow of a user authentication system according to a second embodiment.
FIG. 10 is an example of display in the second embodiment.
FIG. 11 is a functional block diagram showing a configuration of a user authentication system according to another embodiment.
FIG. 12 is a diagram showing an operation flow of a user authentication system according to a third embodiment.

### Description of Embodiments

Hereinafter, embodiments of the present invention will be described in detail with reference to the drawings.

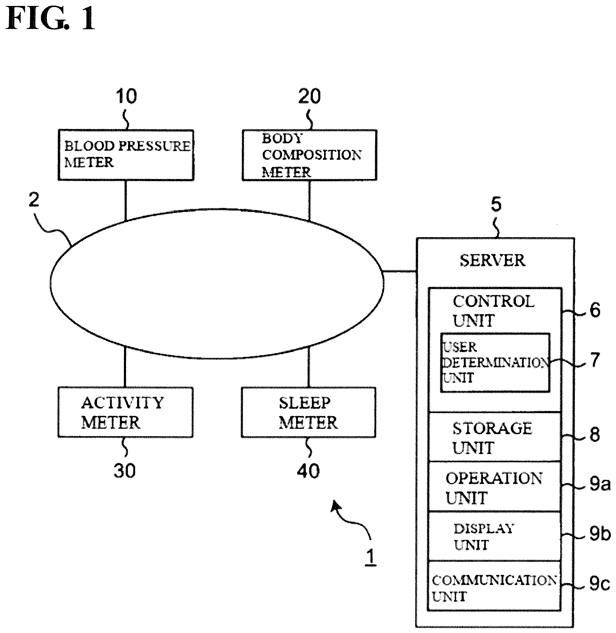

FIG. 1 shows an example of a configuration for when a user authentication system 1 of the present invention, which performs authentication of a person (hereinafter referred to as a user) whose bodily information is acquired by using a bodily information acquisition apparatus, is used on a network 2. The user authentication system 1 includes a blood pressure meter 10, a body composition meter 20, an activity meter 30, and a sleep meter 40, which function as bodily information acquisition apparatuses, as well as a server 5, and the network 2. Bodily information acquisition apparatuses such as the blood pressure meter 10 are configured to be able to mutually communicate with the server 5 by wire or wirelessly via the network 2. The network 2 is an open network (i.e., the Internet) or a network that is used within a specific organization (i.e., an intranet). Access to the server 5 is restricted by a requirement to input a user registration number and a password, thus ensuring the security of the server 5.

The server 5 includes a control unit 6, a user determination unit 7, a storage unit 8, an operation unit 9a, a display unit 9b, and a communication unit 9c. The control unit 6 includes a CPU (Central Processing Unit) and auxiliary circuits thereof, controls the units of the server 5, executes pre-determined processing in accordance with programs and data stored in the storage unit 8, processes data input from the operation unit 9a and the communication unit 9c, stores the processed data in the storage unit 8, displays the processed data on the display unit 9b, and causes the processed data to be output from the communication unit 9c.

The storage unit 8 of the server 5 includes a RAM (Random Access Memory) that is used as a work region needed for the control unit 6 to execute programs, and a ROM (Read Only Memory) for storing basic programs to be executed by the control unit 6. A program for performing determination of individual authentication is installed in the storage unit 8. Also, data obtained by adding information of the measurement date/time, an apparatus identification number, and a user identification number to measured new bodily information so as to associate them with each other is stored as a database in the storage unit 8. Furthermore, a database for user registration information, in which a registered user is associated with an apparatus used by the user, is stored in the storage unit 8. Note that a magnetic disk (HD (Hard Disk), FD (Flexible Disk)), an optical disk (CD (Compact Disc), DVD (Digital Versatile Disk), BD (Blu-ray Disc)), a magneto-optical disk (MO), a semiconductor memory (memory card, SSD (Solid State Drive)), or the like may be used as a storage medium for an auxiliary storage apparatus for assisting the storage region of the storage unit 8. Although the storage unit 8 is shown as a constituent element of the server 5, the storage unit 8 can also have a different configuration in which it is independent of the server 5. Functions of the storage unit 8, for example, can also be carried out by the storage units of the bodily information acquisition apparatuses such as the later-described blood pressure meter 10 or by a storage unit of an external information processing terminal.

The user determination unit 7 of the server 5 checks whether or not there is a logical discrepancy between new bodily information (e.g., a blood pressure value) acquired by a certain bodily information acquisition apparatus (e.g., the blood pressure meter 10) and other bodily information (e.g., sleep state such as bedtime and getting-up time) acquired by a bodily information acquisition apparatus (e.g., the sleep meter 40) other than the certain bodily information acquisition apparatus, and thereby determines whether or not the new bodily information (e.g., the blood pressure value) acquired by the certain bodily information acquisition apparatus (e.g., the blood pressure meter 10) relates to a certain user. In FIG. 1, the user determination unit 7 is included as part of the control unit 6, but the user determination unit 7 may also have a separate configuration in which it is independent of the control unit 6.

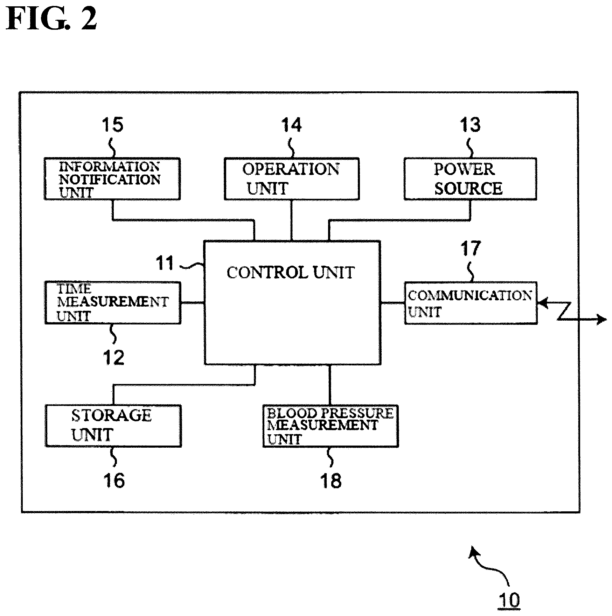

As shown in FIG. 2, the blood pressure meter 10 includes a control unit 11, a time measurement unit 12, a power source 13, an operation unit 14, an information notification unit 15, a storage unit 16, a communication unit 17, and a blood pressure measurement unit 18. That is, the blood pressure meter 10 measures a blood pressure value and a pulse rate as bodily information.

The control unit 11 includes a CPU (Central Processing Unit) and auxiliary circuits thereof, controls the units of the blood pressure meter 10, and executes various types of processing in accordance with the programs and data stored in the storage unit 16. That is, the control unit 11 processes data input from the operation unit 14 and the communication unit 17, stores the processed data in the storage unit 16, uses the information notification unit 15 to perform notification of the processed data, and causes the processed data to be output from the communication unit 17.

Due to the CPU executing a program, the control unit 11 performs control such that blood pressure values (systolic blood pressure and diastolic blood pressure) and a pulse rate of a certain user are measured by the blood pressure measurement unit 18. The control unit 11 adds information of the measurement date/time measured by the time measurement unit 12, information of the apparatus identification number, and information of the user identification number, all of which will be described later, to the information of the measured blood pressure value and pulse rate so as to associate them with each other, and thereby groups them into data relating to the blood pressure value and pulse rate at a certain measurement date/time for a certain user. The control unit 11 performs control such that notification of the measured blood pressure values and pulse rate, the measurement date/time, the user name, or the like is performed by the information notification unit 15. The control unit 11 performs control such that the data relating to the blood pressure values and pulse rate at a certain measurement date/time for a certain user is stored in a time series in the storage unit 16.

The time measurement unit 12 acquires the information of the measurement date/time by measuring the date/time when measurement of the blood pressure values and pulse rate was performed by the blood pressure measurement unit 18. For example, a clock built into the control unit 11 can also be used as the time measurement unit 22.

The storage unit 16 includes a RAM (Random Access Memory) that is used as a work region needed for the control unit 11 to execute programs, and a ROM (Read Only Memory) for storing basic programs to be executed by the control unit 11. A semiconductor memory (memory card, SSD (Solid State Drive)) or the like can be used as a storage medium for an auxiliary storage apparatus for assisting the storage region of the storage unit 16. The data of the measured blood pressure values and pulse rates is stored in the storage unit 16 by the control unit 11 for each user (for each user identification number) in a time series. The storage unit 16 can store unique information of the users (sex, age, height) as well. For example, the blood pressure meter 10 can register data and unique information for multiple users, and can select a user due to a user registration number being designated using the operation unit 14.

The operation unit 14 is constituted by a switch or a touch panel, for example, and inputs operation signals corresponding to the content of operations performed by the user to the control unit 11.

The information notification unit 15 is a display device (e.g., an LCD (Liquid Crystal Display), EL (Electroluminescence) display, or the like) or an audio generator (e.g., a speaker). The information notification unit 15 is controlled by the control unit 11 to display acquired data, warning messages, and messages prompting re-measurement, for example, on the screen of a display device or to generate audio of acquired data, warning messages, and messages prompting re-measurement using an audio generator, and thereby notify a certain user of the possibility that the acquired data does not belong to the certain user.

The blood pressure measurement unit 18 includes a cuff, a pump, a valve, and a pressure sensor, for example. The blood pressure measurement unit 18 is configured such that the cuff is wrapped around a measurement site of a user in advance, and the pump and valve are controlled so as to increase the pressure inside the cuff (cuff pressure) to be greater than the systolic blood pressure, whereafter the pressure is gradually reduced, and the systolic blood pressure, diastolic blood pressure, and pulse rate are measured using a pressure sensor.

The communication unit 17 is for performing communication with an external information processing terminal (not shown). For example, the communication unit 17 is constituted by a communication interface such as USB (Universal Serial Bus) for performing connection by wire with the external information processing terminal, or FeLiCa (registered trademark) or Bluetooth (registered trademark), which are used for NFC (Near Field Communication) for performing wireless communication with the external information processing terminal.

The external information processing terminal connected to the blood pressure meter 10 via the communication unit 17 of the blood pressure meter 10 is, for example, a PC (Personal Computer), a smartphone, a tablet, a PDA (Personal Digital Assistant), or the like. Note that the external information processing terminal can include at least a control unit, a power source, an operation unit, an information notification unit, a storage unit, and a communication unit. Note that in the case of using a configuration in which the blood pressure meter 10 itself includes the function of communicating with an external apparatus, the blood pressure meter 10 can perform communication with the external server 5 directly via the network 2.

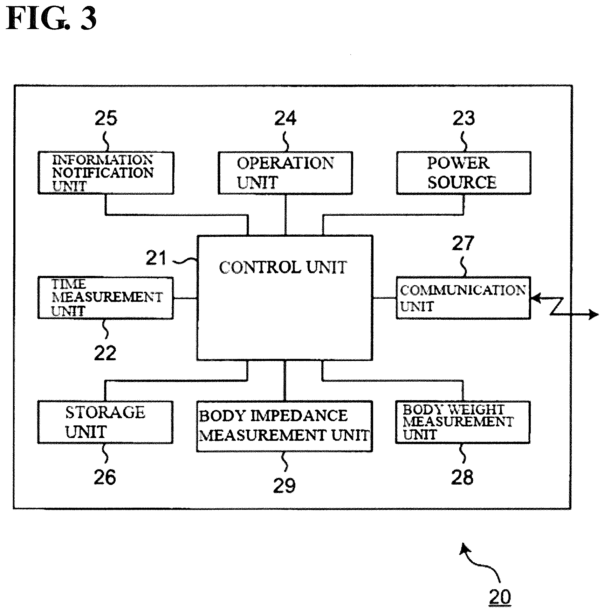

As shown in FIG. 3, a body composition meter 20 includes a control unit 21, a time measurement unit 22, a power source 23, an operation unit 24, an information notification unit 25, a storage unit 26, a communication unit 27, a weight measurement unit 28, and a body impedance measurement unit 29. That is, the body composition meter 20 measures a body weight value and a body composition as bodily information.

The body weight measurement unit 28 is a load sensor that measures the body weight of a user by, for example, sensing a change in a resistance value detected by a strain gauge. The body impedance measurement unit 29 measures the body impedance value of a user by applying a current to the body of the user from a current-applying electrode (not shown) for measuring the body impedance value of a user, and detecting the voltage with a voltage-detecting electrode (not shown).

Based on the measured body weight value and body impedance value and the user information unique to the measured user (age, height, sex, etc.), the control unit 21 calculates the body composition, and the calculated body composition expresses the percentage or mass of components composing the body (structures constituting the body) and is, for example, a body fat percentage, a muscle percentage, a lean body mass, a body fat mass, a muscle mass, a bone mass, and a body water mass.

The control unit 21 adds the information of the measurement date/time acquired by the time measurement unit 22, the information of the apparatus identification number, and the information of the user identification number to the information of the measured body weight value and body composition so as to associate them with each other, and thereby groups them into data relating to the body weight value and body composition at a certain measurement date/time for a certain user. The control unit 21 performs control such that notification of the measured body weight value and body composition, measurement date/time, user name, or the like is performed by the information notification unit 25. The control unit 21 performs control such that data relating to the body weight value and body composition at a certain measurement date/time for a certain user is stored in a time series in the storage unit 26.

Note that the functions of the control unit 21, the time measurement unit 22, the power source 23, the operation unit 24, the information notification unit 25, the storage unit 26, and the communication unit 27 in the body composition meter 20 are substantially the same as the respective functions of the control unit 11, the time measurement unit 12, the power source 13, the operation unit 14, the information notification unit 15, the storage unit 16, and the communication unit 17 of the blood pressure meter 10. Therefore, detailed description thereof will not be included here.

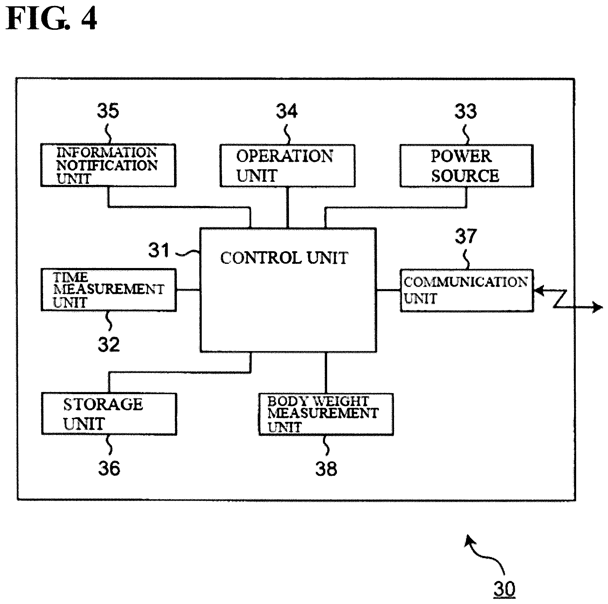

As shown in FIG. 4, the activity meter 30 includes a control unit 31, a time measurement unit 32, a power source 33, an operation unit 34, an information notification unit 35, a storage unit 36, a communication unit 37, and a bodily movement measurement unit 38. That is, the activity meter 30 detects bodily movement of a user in various activity scenes, such as walking, housework, desk work, or jogging, using the bodily movement measurement unit 38, and measures the number of steps and activity amount of the user, the number of steps and activity amount being measured as bodily information. Note that the activity meter 30 can also specialize as a pedometer for measuring only the number of steps of the user.

The bodily movement measurement unit 38 is preferably a three-axis acceleration sensor that is constituted by an acceleration sensor that can detect acceleration of at least one axis of bodily movement that occurs due to walking, daily life activities, and the like and can acquire a larger amount of information relating to the bodily movement of the user. The acceleration signal is obtained from the bodily movement measurement unit 38. Note that by furthermore including an air pressure sensor, the bodily movement measurement unit 38 can discern a state of walking upstairs and obtain the number of steps and activity amount at that time more accurately.

The control unit 31 adds the information of the measurement date/time acquired by the time measurement unit 32, the information of the apparatus identification number, and the information of the user identification number to the information of the measured number of steps and activity amount so as to associate them with each other, and thereby groups them into data relating to the number of steps and activity amount at a certain measurement date/time for a certain user. The control unit 31 performs control such that notification of the measured number of steps and activity amount, the measurement date/time, the user name, or the like is performed by the information notification unit 35. The control unit 31 performs control such that the data relating to the number of steps and activity amount at the certain measurement date/time for the certain user is stored in a time series in the storage unit 36.

Note that the functions of the control unit 31, the time measurement unit 32, the power source 33, the operation unit 34, the information notification unit 35, the storage unit 36, and the communication unit 37 of the activity meter 30 are substantially the same as the respective functions of the control unit 11, the time measurement unit 12, the power source 13, the operation unit 14, the information notification unit 15, the storage unit 16, and the communication unit 17 of the blood pressure meter 10. Therefore, detailed description thereof will not be included here.

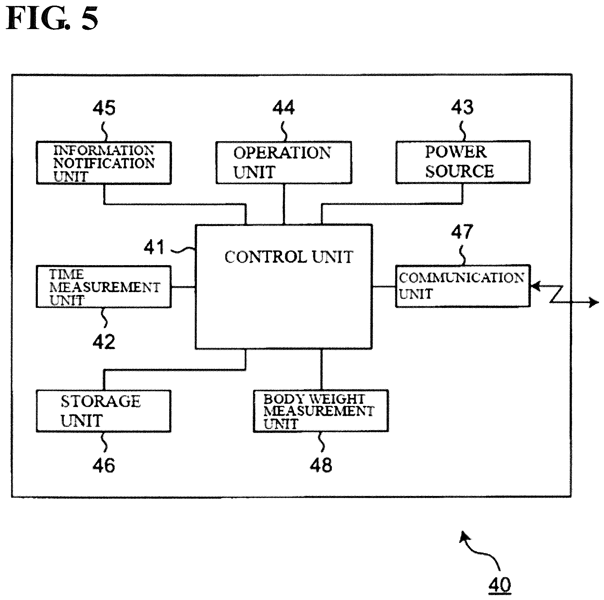

As shown in FIG. 5, the sleep meter 40 includes a control unit 41, a time measurement unit 42, a power source 43, an operation unit 44, an information notification unit 45, a storage unit 46, a communication unit 47, and a bodily movement measurement unit 48. That is, the sleep meter 40 uses the bodily movement measurement unit 48 to detect movement of bedding caused by a sleeping user moving, and measures the sleeping state, such as the sleeping time from bedtime to getting-up time, sleep depth, and the like as bodily information.

The bodily movement measurement unit 48 is preferably a three-axis acceleration sensor that is constituted by an acceleration sensor that can detect the acceleration of at least one axis of movement of bedding caused by a sleeping user moving, and can detect a larger amount of information relating to the bodily movement of the user. The acceleration signal is obtained from the bodily movement measurement unit 48.

The control unit 41 adds the information of the measurement date/time acquired by the time measurement unit 42, the information of the apparatus identification number, and the information of the user identification number to the information of the measured sleep state so as to associate them with each other, and thereby groups them into data relating to the sleep state at a certain measurement date/time for a certain user. The control unit 41 performs control such that notification of the measured sleep state, the measurement date/time, the user name, or the like is performed by the information notification unit 45. The control unit 41 performs control such that data relating to the sleep state at the certain measurement date/time for the certain user is stored in a time series in the storage unit 46.

Note that the functions of the control unit 41, the time measurement unit 42, the power source 43, the operation unit 44, the information notification unit 45, the storage unit 46, and the communication unit 47 of the sleep meter 40 are substantially the same as the respective functions of the control unit 11, the time measurement unit 12, the power source 13, the operation unit 14, the information notification unit 15, the storage unit 16, and the communication unit 17 of the blood pressure meter 10, and therefore detailed description thereof will not be included here.

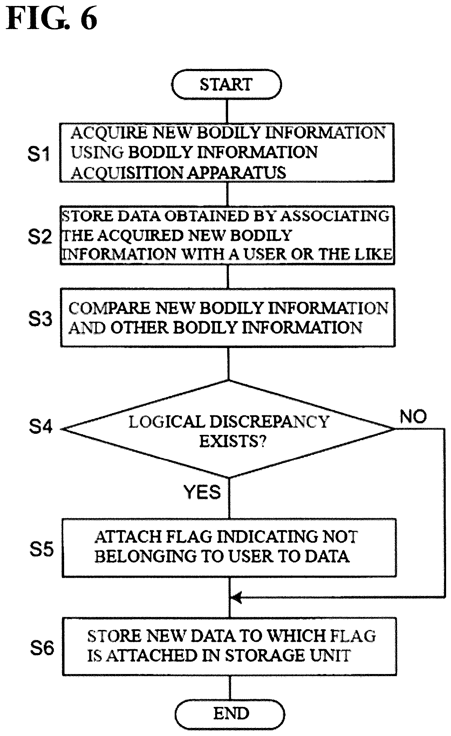

Next, a basic operation of the user authentication system 1 will be described with reference to FIGS. 1 and 6.

First, multiple users including a certain user use the bodily information acquisition apparatuses such as the blood pressure meter 10, whereby respective pieces of bodily information such as blood pressure values for the multiple users including the certain user are acquired (step S1).

The information of the measurement date/time, the information of the apparatus identification number of the bodily information acquisition apparatus used by the user, and the information of the user identification number of the user who used the bodily information acquisition apparatus are added to the acquired bodily information such as the blood pressure value such that they are associated with each other and are thereby grouped into data relating to the certain bodily information at the certain measurement date/time for the certain user, and the data is stored in a time series in the storage unit of the bodily information acquisition apparatus (step S2).

When the certain bodily information acquisition apparatus is connected to the network 2, the data relating to the certain bodily information stored in the storage unit of the certain bodily information acquisition apparatus is sent to the server 5 as a batch. New data relating to the certain bodily information transmitted to the server 5 is stored temporarily in the storage unit 8 of the server 5 as new bodily information. Similarly, when another bodily information acquisition apparatus such as the sleep meter 40 is connected to the network 2, other bodily information is transmitted to the server 5 and temporarily stored in the storage unit 8 of the server 5.

New data temporarily stored in the storage unit 8 of the server 5 includes information of the user identification number of the user who used the certain bodily information acquisition apparatus, and therefore the certain user is specified as one of the users registered in the database for the user registration information. According to the control unit 6, based on the database for the user registration information, another bodily information acquisition apparatus used by the certain user who was specified is searched for, and the other bodily information acquisition apparatus used by the specified certain user is specified.

The user determination unit 7 of the server 5 individually compares the temporarily-stored new bodily information and the other bodily information acquired by the other bodily information acquisition apparatus such as the sleep meter 40 (step S3). Then, the user determination unit 7 determines whether or not there is a logical discrepancy between the new bodily information and the other bodily information (step S4). As a result, when it is determined that there is no logical discrepancy between the new bodily information and the other bodily information, the new bodily information is stored in the storage unit 8 of the server 5 as new data of certain bodily information for a certain user.

If the user determination unit 7 determines that there is a logical discrepancy between the new bodily information and the other bodily information due to the fact that acquisition of the new bodily information and other bodily information is substantially impossible from a temporal and spatial (positional) perspective, and cannot happen in reality, the control unit 6 attaches a flag (attribute information) indicating that the new bodily information does not belong to the certain user to the corresponding data location in the new data (step S5).

The new data to which the flag indicating not belonging to the certain user is attached is stored in the storage unit 8 of the server 5 as new data of the certain bodily information for the certain user (step S6). Then, when the registration processing of the new data for the certain bodily information for the certain user ends, the new data for the certain bodily information for the certain user is displayed on the display unit of an information processing terminal connected to the network 2, thereby making it available for the certain user or the like to browse.

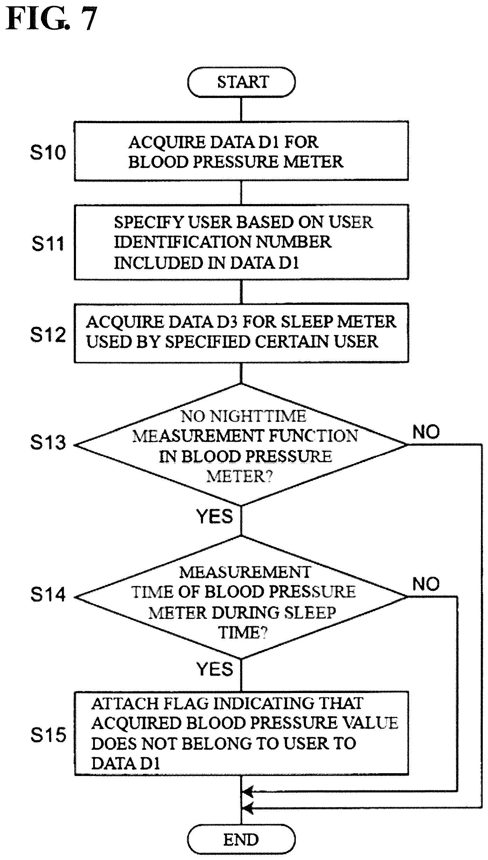

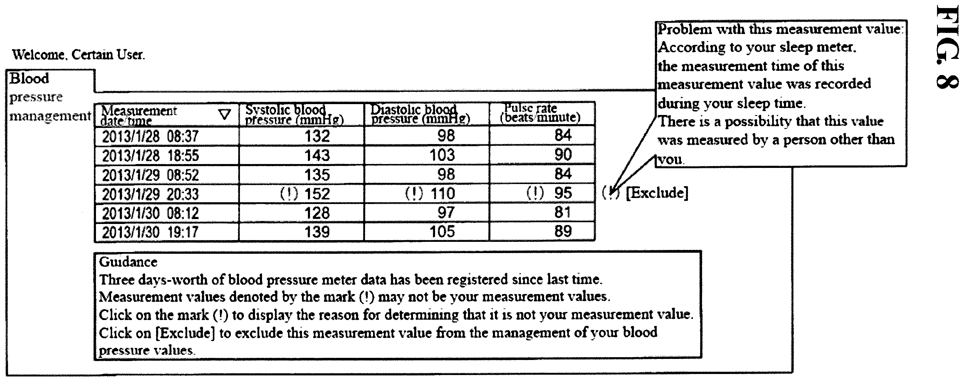

Next, a case in which the blood pressure meter 10 is used as the certain bodily information acquisition apparatus and the sleep meter 40 is used as the other bodily information acquisition apparatus will be described as a first embodiment with reference to FIGS. 7 and 8. Note that the first embodiment is an example for describing an operation of the user authentication system 1 in a manner that is easy to understand, and is naturally not limited to the specific numeric values and the like described in the first embodiment.

In FIG. 7, multiple users including the certain user use the blood pressure meter 10, and thus blood pressure values for the multiple users including the certain user are acquired.

The information of the measurement date/time, the information of the apparatus identification number of the blood pressure meter 10 used by the user, and the information of the user identification number of the certain user are added to the information of the acquired blood pressure value such that they are associated with each other and are thereby grouped into new data D1 relating to the blood pressure value at the certain measurement date/time for the certain user, and the new data D1 shown in Table 1 as an example is stored in a time series in the storage unit 16 of the blood pressure meter 10 (step S10).

**Table 1**

| Apparatus identification number | User identification number | Measurement date/time | | Systolic blood pressure | Diastolic blood pressure | Pulse rate |
|---|---|---|---|---|---|---|
| A123 | 1 | 2013/1/28 | 8:37 | 132 | 98 | 84 |
| A123 | 1 | 2013/1/28 | 18:55 | 143 | 103 | 90 |
| A123 | 1 | 2013/1/29 | 8:52 | 135 | 98 | 84 |
| A123 | 1 | 2013/1/29 | 20:33 | 152 | 110 | 95 |
| A123 | 1 | 2013/1/30 | 8:12 | 128 | 97 | 81 |
| A123 | 1 | 2013/1/30 | 19:17 | 139 | 105 | 89 |

When the blood pressure meter 10 is connected to the network 2, the new data D1 relating to the blood pressure value stored in the storage unit 16 of the blood pressure meter 10 is transmitted as a batch to the server 5. The new data D1 relating to the blood pressure value transmitted to the server 5 is stored temporarily in the storage unit 8 of the server 5.

The new data D1 temporarily stored in the storage unit 8 of the server 5 includes information of the user identification number of the certain user who used the blood pressure meter 10. Therefore, among the users registered in the database for the user registration information shown in Table 2 as an example, the user to which the certain user corresponds is searched for, and it is specified that the user registration number for the certain user is U003 (step S11). Hereinafter, the thus-specified certain user U003 will be referred to as specified user U003.

**Table 2**

| User registration number | Apparatus name | Apparatus identification number | User identification number |
|---|---|---|---|
| U003 | Sleep meter | S023 | NA |
| U003 | Activity meter | P723 | NA |
| U003 | Body composition meter | F123 | 4 |
| U003 | Blood pressure meter | A123 | 1 |
| U007 | Blood pressure meter | A123 | 2 |
| U012 | Blood pressure meter | A356 | 1 |

Based on the database for the user registration information shown in Table 2 as an example, the control unit 6 searches for another bodily information acquisition apparatus used by the specified user U003. For example, according to the database for the user registration information shown in Table 2, the specified user U003 has registered and used four bodily information acquisition apparatuses. As a result of searching, the sleep meter 40 is specified as another bodily information acquisition apparatus used by the specified user U003.

Similarly to the blood pressure meter 10, when the sleep meter 40 is connected to the network 2, the data D3 for the sleep state (date/time of going to bed and date/time of getting up) shown in FIG. 3 as an example is transmitted to the server 5 and stored temporarily (i.e., acquired) in the storage unit 8 of the server 5 (step S12).

**Table 3**

| Apparatus identification number | Date/time of going to bed | | Date/time of getting up | |
|---|---|---|---|---|
| S023 | 2013/1/28 | 21:35 | 2013/1/29 | 6:12 |
| S023 | 2013/1/29 | 20:01 | 2013/1/30 | 6:56 |
| S023 | 2013/1/30 | 20:52 | 2013/1/31 | 7:07 |

The user determination unit 7 of the server 5 individually compares the new data D1 relating to the blood pressure values and other data D3 relating to the sleep states (date/time of going to bed and date/time of getting up). Then, the user determination unit 7 determines whether or not there is a logical discrepancy between the new data D1 relating to the blood pressure value and the other data D3 relating to the sleep state (date/time of going to bed and date/time of getting up) (steps S13 and S14). For example, in step S13, it is determined whether or not the blood pressure meter 10 is provided with a nighttime measurement function, and in step S14, it is determined whether or not the date/time of measurement performed by the blood pressure meter 10 is within range of the sleep time of the specified user U003.

It is determined whether or not the specified user U003 has performed blood pressure measurement during nighttime, which is a sleep time, despite the fact that the blood pressure meter 10 is not provided with the nighttime measurement function. For example, with a focus on the time slot of 20:00 on 2013/1/29, the specified user U003 is in bed at 20:01 despite the fact that the specified user U003 performed blood pressure measurement at 20:33. Performing blood pressure measurement using the blood pressure meter 10, which does not include the nighttime measurement function, is substantially impossible and cannot happen in reality. Thus, from a temporal perspective, the acquisition of the new data D1 relating to the blood pressure value for the specified user U003 and the acquisition of the other data D3 relating to the sleep states (date/time of going to bed and date/time of getting up) is substantially impossible and cannot happen in reality. Therefore, a logical discrepancy exists between the two. Accordingly, since there is a logical discrepancy between the new data D1 relating to the blood pressure value and the other data D3 relating to the sleep state (date/time of going to bed and date/time of getting up), the user determination unit 7 determines that there is a logical discrepancy, and the control unit 6 attaches a flag indicating that the new data D1 relating to the blood pressure value does not belong to the specified user U003 to the corresponding data location in the new data D1 relating to the blood pressure value (step S15).

The control unit 6 stores the new data D1 to which the flag indicating not belonging to the specified user U003 is attached in the storage unit 8 of the server 5 as the new data D1 relating to the blood pressure value for the specified user U003. Also, in steps S13 and S14, if it is determined that there is no logical discrepancy between the new data D1 relating to the blood pressure value and the other data D3 relating to the sleep state (date/time of going to bed and date/time of getting up), the new data D1 relating to the blood pressure value for the specified user U003 is stored in the storage unit 8 of the server 5 as a database.

According to the above procedure, when the registration processing of the new data D1 relating to the blood pressure value for the specified user U003 ends, the registered new data D1 is subjected to data processing by the control unit 6 of the server 5, whereafter it is displayed on the display unit of the information processing terminal connected to the network 2 in the mode of a table of numerical values or a graph, thereby making it available for the specified user U003 or the like to browse.

The registered new data D1 is displayed on the display unit of the information processing terminal in the mode shown in FIG. 8 for example. In the display example shown in FIG. 8, if it is determined that there is a logical discrepancy regarding a data location, display for making an inquiry about that data location to the specified user U003 is performed. In the display unit, when the check location [Exclude] is selected, the data location is not only erased from the display unit, but is excluded from the calculation of an average value and the number of instances of measurement. Note that in order to make it possible to cancel selection of the check location [Exclude] caused by erroneous operation, it is possible to use a configuration in which only erasing from the display unit, and exclusion from calculation of the average value and counting of instances of measurement are performed, and storage as a database is maintained. It is also possible to use a configuration in which the data location is erased from the display unit and excluded from the calculation of the average value and the counting of the number of instances of measurement automatically by the control unit 6 of the server 5 without making an inquiry to the specified user U003.

Accordingly, in the user authentication system 1 of the above-described first embodiment, user recognition can be performed with a higher accuracy by checking whether or not a logical discrepancy exists between the new data D6 relating to the blood pressure value and the other data D3 relating to the sleep state (date/time of going to bed and date/time of getting up)

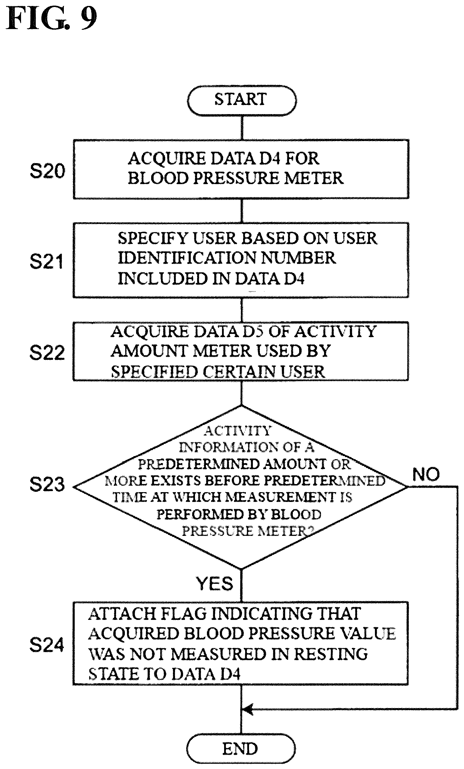

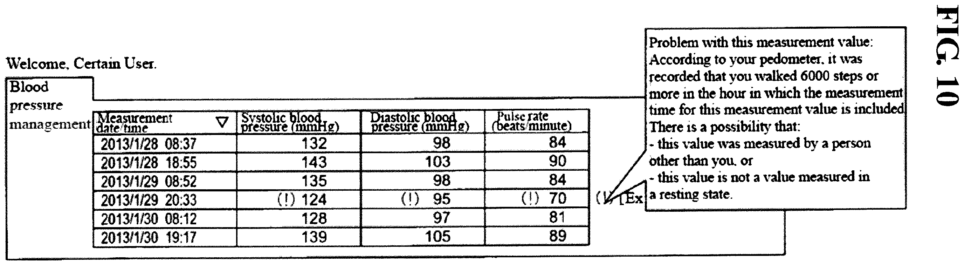

Next, with reference to FIGS. 9 and 10, as a second embodiment, a case will be described in which the blood pressure meter 10 is used as the certain bodily information acquisition apparatus, and the activity meter 30 is used as the other bodily information acquisition apparatus. Note that the second embodiment is also an example for describing an operation of the user authentication system 1 in a manner that is easy to understand, and it is naturally the case that there is no limitation to the specific numerical values and the like shown in the second embodiment.

In FIG. 9, multiple users including the certain user use the blood pressure meter 10, and thereby blood pressure values for the multiple users including the certain user are acquired.

Information of the measurement date/time, information of the apparatus identification number of the blood pressure meter 10 used by the users, and information of the user identification number of the certain user are added to the information of an acquired blood pressure value such that they are associated with each other and are thereby grouped into new data D4 relating to the blood pressure value at a certain measurement date/time for the certain user, and the new data D4 shown in Table 4 as an example is stored in a time series in the storage unit 16 of the blood pressure meter 10 (step S20).

**Table 4**

| Apparatus identification number | User identification number | Measurement date/time | | Systolic blood pressure | Diastolic blood pressure | Pulse rate |
|---|---|---|---|---|---|---|
| A123 | 1 | 2013/1/28 | 8:37 | 132 | 98 | 84 |
| A123 | 1 | 2013/1/28 | 18:55 | 143 | 103 | 90 |
| A123 | 1 | 2013/1/29 | 8:52 | 135 | 98 | 84 |
| A123 | 1 | 2013/1/29 | 20:33 | 124 | 95 | 70 |
| A123 | 1 | 2013/1/30 | 8:12 | 128 | 97 | 81 |
| A123 | 1 | 2013/1/30 | 19:17 | 139 | 105 | 89 |

When the blood pressure meter 10 is connected to the network 2, the new data D4 relating to the blood pressure value stored in the storage unit 16 of the blood pressure meter 10 is transmitted to the server 5 as a batch. The new data D4 relating to the blood pressure values transmitted to the server 5 is stored temporarily in the storage unit 8 of the server 5.

The new data D4 that is temporarily stored in the storage unit 8 of the server 5 includes information of the user identification number of the certain user who used the blood pressure meter 10. Therefore, among the users registered in the database of the user registration information shown in Table 5 as an example, the user corresponding to the certain user is searched for, and it is specified that the user registration number for the certain user is U003 (step S21).

**Table 5**

| User registration number | Apparatus name | Apparatus identification number | User identification number |
|---|---|---|---|
| U003 | Sleep meter | S023 | NA |
| U003 | Activity meter | P723 | NA |
| U003 | Body composition meter | F123 | 4 |
| U003 | Blood pressure meter | A123 | 1 |
| U009 | Activity meter | P781 | 15 |
| U012 | Blood pressure meter | A356 | 1 |

Based on the database for user registration information shown in Table 5 as an example, the control unit 6 searches for another bodily information acquisition apparatus used by the specified user U003. For example, according to the database for the user registration information shown in Table 5, the specified user U003 has registered and used four bodily information acquisition apparatuses. As a result of the searching, the activity meter 30 is specified as the other bodily information acquisition apparatus used by the specified user U003.

Similarly to the blood pressure meter 10, when the activity meter 30 is connected to the network 2, the data D5 for the activity amount (number of steps) shown in Table 6 as an example is transmitted to the server 5 and stored temporarily (i.e., acquired) in the storage unit 8 of the server 5 (step S22).

**Table 6**

| Apparatus identification number | Measurement date | Measurement time slot | Number of steps | Exercise intensity |
|---|---|---|---|---|
| P723 | 2013/1/29 | *** | *** | *** |
| P723 | 2013/1/29 | *** | *** | *** |
| P723 | 2013/1/29 | *** | *** | *** |
| P723 | 2013/1/29 | 18 | 15 | 1 |
| P723 | 2013/1/29 | 19 | 3123 | 3 |
| P723 | 2013/1/29 | 20 | 6568 | 5 |
| P723 | 2013/1/29 | 21 | 512 | 2 |
| P723 | 2013/1/29 | 22 | 10 | 1 |
| P723 | 2013/1/29 | 23 | 13 | 1 |
| P723 | 2013/1/29 | 24 | 0 | 0 |

In FIG. 9, the user determination unit 7 of the server 5 individually compares the new data D4 relating to the blood pressure value and the other data D5 relating to the activity amount (number of steps). Then, the user determination unit 7 determines whether or not a logical discrepancy exists between the new data D4 relating to the blood pressure value and the other data D5 relating to the activity amount (number of steps) (step S23). For example, in step S23, it is determined whether or not there is activity information of a predetermined amount or more before a predetermined time measured by the blood pressure meter 10. For example, with a focus on the time slot of 20:00 on 2013/1/29, the specified user U003 walked 6568 steps/hour despite the fact that blood pressure measurement was being performed by the blood pressure meter 10. That is a walking speed of 1.8 steps/second. In practice, it is difficult to perform blood pressure measurement while walking at such a speed, and even in the unlikely event that the blood pressure measurement was performed, the blood pressure value would not be reliable because blood pressure measurement was not performed while at rest. Accordingly, there is a logical discrepancy between the new data D4 relating to the blood pressure value and the other data D5 relating to the activity amount (number of steps), and therefore the user determination unit 7 determines that there is a logical discrepancy. Then, the control unit 6 attaches a flag indicating that the new data D4 relating to the blood pressure value was not measured in a resting state to the corresponding data location in the new data D4 relating to the blood pressure value (step S24).

The new data D4, to which the control unit 6 attached the flag indicating not having been measured in a resting state, is stored in the storage unit 8 of the server 5 as the new data D4 relating to the blood pressure value for the specified user U003. Also, if it is determined in step S23 that there is no logical discrepancy between the new data D4 relating to the blood pressure value and the other data D5 relating to the activity amount (number of steps), the new data D4 relating to the blood pressure value for the specified user U003 is stored as a database in the storage unit 8 of the server 5.

With the above procedure, when the registration processing for the new data D4 relating to the blood pressure value for the specified user U003 ends, the registered new data D4 is subjected to data processing by the control unit 6 of the server 5, whereafter it is displayed on the display unit of the information processing terminal connected to the network 2 in a mode of a table of numerical values or a graph, thereby making it available for the specified user U003 or the like to browse.

The registered new data D4 is displayed on the display unit of the information processing terminal in the mode shown in FIG. 10 for example. In the display example of FIG. 10, if it is determined that there is a logical discrepancy regarding the data location, a message prompting confirmation of the data location, a message to the effect that there is a possibility that the data location was measured in the non-resting state, and the like are displayed to the specified user U003. If the check location [Exclude] is selected on the display unit, the corresponding data location is not only erased from the display portion, but is excluded from the calculation of the average value and the number of instances of measurement. Note that in order to make it possible to cancel selection of the check location [Exclude] caused by erroneous operation, it is possible to use a configuration in which only erasing from the display unit, and exclusion from calculation of the average value and counting of the number of instances of measurement are performed, and storage as a database is maintained. It is also possible to use a configuration in which the data location is erased from the display unit and excluded from the calculation of the average value and the counting of the number of instances of measurement automatically by the control unit 6 of the server 5 without making an inquiry to the specified user U003.

Accordingly, in the user authentication system 1 of the above-described second embodiment as well, user recognition can be performed with higher accuracy by checking whether or not a logical discrepancy exists between the new data D6 relating to the blood pressure value and the other data D5 relating to the activity amount (number of steps).

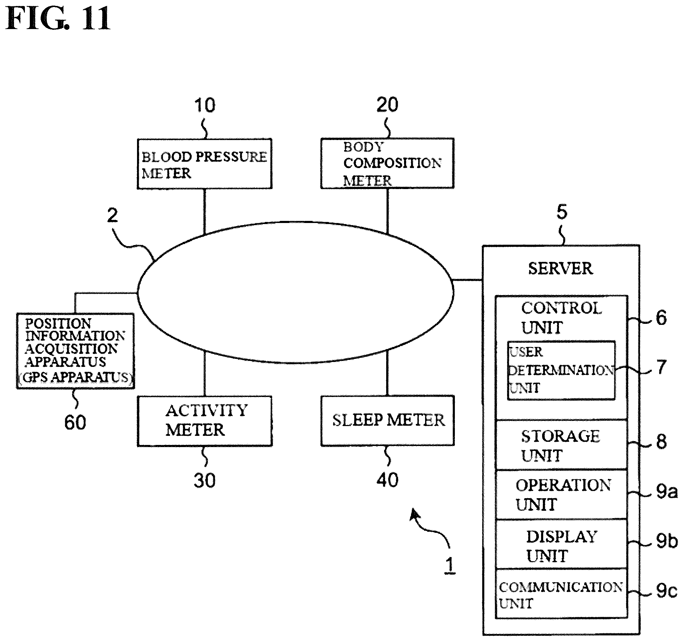

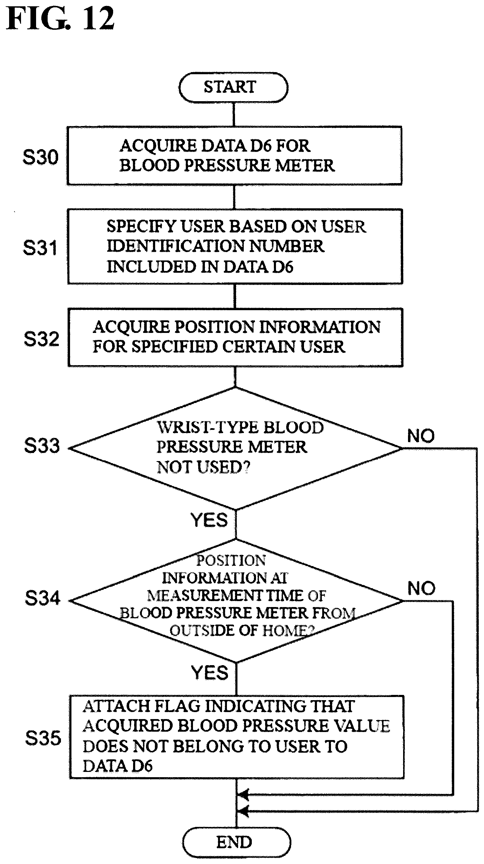

Although another embodiment will be described with reference to FIGS. 11 and 12 below, description of portions that are the same as in the above-described embodiments will not be included.

FIG. 11 shows an example of a configuration for when the user authentication system 1 according to another embodiment is used on the network 2. The user authentication system 1 includes the blood pressure meter 10, the body composition meter 20, the activity meter 30, and the sleep meter 40, which function as bodily information acquisition apparatuses, a position information acquisition apparatus 60, the server 5, and the network 2. The bodily information acquisition apparatuses such as the blood pressure meter 10 and the position information acquisition apparatus 60 are each configured to be able to mutually communicate by wire or wirelessly with the server 5 via the network 2.

The position information acquisition apparatus 60 includes a position information acquisition unit, a control unit, a power source, an operation unit, a time measurement unit, a storage unit, and a communication unit. The position information acquisition apparatus 60 is a GPS (global positioning system) apparatus that has, for example, a GPS reception unit, a control unit, a power source, an operation unit, a storage unit, and a communication unit. In the GPS apparatus, the GPS reception unit receives radio waves from multiple GPS satellites, and the control unit calculates the current position information according to GPS positioning and specifies the current position information (e.g., latitude and longitude, location registered in advance, such as the home or workplace of the certain user). Note that information of the date/time is also included in the current position information acquired using GPS positioning, and therefore the time measurement unit can be omitted in the GPS apparatus.

As the position information acquisition apparatus 60 that does not use GPS, it is also possible to use a mobile communication terminal that communicates with a base station of a communication network, such as a mobile phone, a PHS phone, or a smartphone. The mobile communication terminal includes a communication unit also used as a base station position information acquisition unit, a control unit, a power source, an operation unit, a time measurement unit, and a storage unit, and the base station position information acquisition unit (communication unit) acquires position information of the terminal from a position information providing means, such as a base station. The mobile communication terminal acquires position information of the base station, and therefore has a lower positional accuracy than a terminal that uses GPS, but even if the mobile communication terminal is inside a building, between buildings with many stories, in an underground shopping center, or the like, where radio waves from a GPS satellite do not reach, it is possible to obtain approximate position information of the mobile communication terminal. Accordingly, a mobile communication terminal such as that described above can also be used as the position information acquisition apparatus 60 that acquires position information on the certain user. Note that if the mobile communication terminal includes the GPS reception unit, position information obtained using GPS and/or position information obtained using a base station can be used.

Next, a case in which the blood pressure meter 10 is used as the certain bodily information acquisition apparatus and a GPS apparatus is used as the position information acquisition apparatus 60 will be described as a third embodiment with reference to FIG. 12. Note that the third embodiment is an example for describing an operation of the user authentication system 1 in a manner that is easy to understand, and it is naturally the case that there is no limitation to the specific numeral values and the like disclosed in the third embodiment.

In FIG. 12, multiple users including the certain user use the blood pressure meter 10, and thereby the blood pressure values for the multiple users including the certain user are acquired.

Information of the measurement date/time, information of the apparatus identification number of the blood pressure meter 10 used by the user, and information of the user identification number of the certain user are added to the information of the acquired blood pressure value such that they are associated with each other and are thereby grouped together into new data D6 relating to the blood pressure value at a certain measurement date/time for the certain user, and the new data D6 shown in Table 7 as an example is stored as a time series in the storage unit 16 of the blood pressure meter 10 (step S30)

**Table 7**

| Apparatus identification number | User identification number | Measurement date/time | | Systolic blood pressure | Diastolic blood pressure | Pulse rate |
|---|---|---|---|---|---|---|
| A243 | 1 | 2013/1/28 | 8:37 | 132 | 98 | 84 |
| A243 | 1 | 2013/1/28 | 18:55 | 143 | 103 | 90 |
| A243 | 1 | 2013/1/29 | 8:52 | 135 | 98 | 84 |
| A243 | 1 | 2013/1/29 | 20:33 | 152 | 110 | 95 |
| A243 | 1 | 2013/1/30 | 8:12 | 128 | 97 | 81 |
| A243 | 1 | 2013/1/30 | 19:17 | 139 | 105 | 89 |

When the blood pressure meter 10 is connected to the network 2, the new data D6 relating to the blood pressure value stored in the storage unit 16 of the blood pressure meter 10 is transmitted to the server 5 as a batch. The new data D6 relating to the blood pressure value transmitted to the server 5 is temporarily stored in the storage unit 8 of the server 5.

The new data D6 temporarily stored in the storage unit 8 of the server 5 includes information of the user identification number of the certain user who used the blood pressure meter 10. Therefore, among the users registered in the database for the user registration information shown in Table 8 as an example, the user corresponding to the certain user is searched for, and it is specified that the user registration number for the certain user is U003 (step S31).

**Table 8**

| User registration number | Apparatus name | Apparatus identification number | User identification number |
|---|---|---|---|
| U003 | GPS apparatus | G963 | NA |
| U003 | Activity meter | P723 | NA |
| U003 | Body composition meter | F123 | 4 |
| U003 | Blood pressure meter | A243 | 1 |
| U007 | Blood pressure meter | A123 | 2 |
| U012 | Blood pressure meter | A356 | 1 |

Based on the database for the user registration information shown in Table 8 as an example, the control unit 6 searches for the position information acquisition apparatus 60 used by the specific user U003. For example, according to the database for the user registration information in Table 8, the specified user U003 has registered and used four bodily information acquisition apparatuses. As a result of searching, the GPS apparatus is specified as the position information acquisition apparatus 60 used by the specified user U003.

Similarly to the blood pressure meter 10, when the GPS apparatus 60 is connected to the network 2, the data D7 for the position information shown in Table 9 as an example is transmitted to the server 5, and is temporarily stored (i.e., acquired) in the storage unit 8 of the server 5 (step S32).

**Table 9**

| Apparatus identification number | Measurement date/time | | Position information |
|---|---|---|---|
| G963 | 2013/1/28 | 21:35 | Home |
| G963 | 2013/1/29 | 20:01 | Restaurant |
| G963 | 2013/1/30 | 20:52 | Workplace |

In FIG. 12, the user determination unit 7 of the server 5 individually compares the new data D6 relating to the blood pressure value and the other data D7 relating to the position information. Also, the user determination unit 7 determines whether or not there is a logical discrepancy between the new data D6 relating to the blood pressure value and the other data D7 relating to the position information (steps S33 and S34). That is, in step S33, it is determined whether or not a wrist-type blood pressure meter 10 was used, and in step S34, it is determined whether or not the position information of the specified user U003 is other than the home of the specified user U003 at the date/time of measurement using the blood pressure meter 10.

For example, with a focus on the time slot of 20:00 on 2013/1/29, the specified user U003 is dining at a restaurant at 20:01, despite the fact that the specified user U003 performed the blood pressure measurement at 20:33. Performing blood pressure measurement at a restaurant using not a wrist-type blood pressure meter 9 but a slightly large blood pressure meter 10 of a type through which the arm is passed is substantially impossible and cannot happen in reality. From a spatial (positional) perspective, the acquisition of the new data D6 relating to the blood pressure value for the specified user U003 and the acquisition of the other data D7 relating to the position information are substantially impossible and cannot happen in reality, and therefore a logical discrepancy exists between the two. Accordingly, because there is a logical discrepancy between the new data D6 relating to the blood pressure value and the other data D7 relating to the position information, the user determination unit 7 determines that there is a logical discrepancy, and the control unit 6 attaches a flag indicating that the new data D6 relating to the blood pressure value does not belong to the specified user U003 to the corresponding data location in the new data D6 relating to the blood pressure value (step S35)

The control unit 6 stores the new data D6, to which the flag indicating not belonging to the specified user U003 is attached, in the storage unit 8 of the server 5 as the new data D6 relating to the blood pressure value for the specified user U003. In steps S33 and S34, if it is determined that there is no logical discrepancy between the new data D6 relating to the blood pressure value and the other data D7 relating to the position information, the new data D6 relating to the blood pressure value for the specified user U003 is stored in the storage unit 8 of the server 5 as a database.

According to the above procedure, when the registration processing for the new data D6 relating to the blood pressure value for the specified user U003 ends, the registered new data D6 is subjected to data processing by the control unit 6 of the server 5, whereafter it is displayed in the mode of a table of numerical values or a graph on the display unit of the information processing terminal connected to the network 2, and thus is made available for the specified user U003 or the like to browse.

Accordingly, even in the user authentication system 1 of the above-described third embodiment, user recognition can be performed at a higher accuracy by checking whether or not there is a logical discrepancy between the new data D6 relating to the blood pressure value and the other data D7 relating to the position information.

Note that in addition to the above description, a body weight meter that measures only the body weight of a user, a thermometer that measures the body temperature of a user, an electrocardiograph that measures current that accompanies movement of the heart muscles of a user, or a blood sugar meter that measures the blood sugar value of the user can also be applied as the bodily information acquisition apparatus in the user authentication system 1. Furthermore, it is possible to use a configuration in which the user authentication system 1 is not provided with the server 5 and the user authentication system 1 is constituted by a bodily information acquisition apparatus including a function similar to that of the server 5.

The above-described user authentication system 1 checks whether or not there is a logical discrepancy based on a relatively short time interval (e.g., in units of minutes or in units of hours) in which the times at which data acquisition was performed are close together or overlap, but it is possible to use a configuration in which it is checked whether or not there is a logical discrepancy based on trends in a relatively long time interval (e.g., day units such as morning, daytime, and evening, week units such as weekdays and weekends, month units such as the beginning of a month, middle of a month, and the end of a month, or year units such as spring, summer, fall, and winter) from the past for the specified user U003, which are accumulated in the database.

The above-described user authentication system 1 checks whether or not there is a logical discrepancy under the assumption that the other bodily information (sleep state, activity amount (number of steps)) and the position information are correct, but it is possible to use a configuration in which the judgment of the user U003 is sought by allowing the specific user U003 to check whether or not the information is correct. It is also possible to check whether or not there is a logical discrepancy by combining multiple pieces of other bodily information and position information.

The above-described embodiments and variations thereof are examples for facilitating understanding of the present invention and are not to be interpreted as being limiting. The technical scope of the present invention is to be defined by the claims. The above-described multiple embodiments can be realized independently of each other, and embodiments can also be combined. The various characteristics of the different embodiments can also be realized independently of each other, and the characteristics of the different embodiments can also be combined.

### Reference Signs List

- 1: User authentication system
- 2: Network
- 5: Server
- 6: Control unit
- 7: User determination unit
- 10: Blood pressure meter
- 20: Body composition meter
- 30: Activity meter
- 40: Sleep meter
- 60: Position information acquisition apparatus (GPS apparatus)

## Claims

1. A user authentication system (1) comprising:
a plurality of types of bodily information acquisition apparatuses (10, 20, 30, 40) configured to acquire different types of bodily information for a certain user;
a control unit (31, 41) configured to generate a group of associated data relating to the bodily information each time new bodily information is measured by a certain bodily information acquisition apparatus, the group of data comprising at least information of the date and time of a measurement, the identification number of the user, the information of the certain apparatus identification number, and the measured bodily information;
a storage unit (8) configured to store the newly generated group of data;
a user determination unit (7) configured to check whether or not there is a logical discrepancy between the newly generated group of data for the user and other previously stored group of data for the same user, said previously stored group of data being associated with a bodily information acquisition apparatus other than said certain bodily information acquisition apparatus, and thereby determine whether or not the newly generated group of data relates to the certain user; **characterized in that** the measured bodily information of said previously stored group of data is of a different type than the bodily information of the newly generated group of data and the logical discrepancy is due to the determination that the acquisition of the newly generated data at the date and time of the newly generated group of data is impossible from a temporal perspective.

2. The user authentication system (1) according to claim 1, wherein
a said storage unit (8) is provided in each of the bodily information acquisition apparatuses (10, 20, 30, 40) and/or is provided independently of the bodily information acquisition apparatuses (10, 20, 30, 40).

3. The user authentication system (1) according to claim 1, wherein
if it is determined by the user determination unit (7) that the newly generated group of data does not relate to the certain user, attribute information indicating that the newly generated group of data does not relate to the certain user is attached to the bodily information of the newly generated group of data.

4. The user authentication system (1) according to claim 1, further comprising
an information notification unit (15, 25, 35, 45) configured to perform notification of the newly generated group of data, information of the certain user, and information relating to the determination result,
wherein if it is determined by the user determination unit (7) that the newly generated group of data does not relate to the certain user, notification of a message relating to the determination result is performed by the information notification unit.

5. The user authentication system (1) according to claim 1, further comprising
an information notification unit (15, 25, 35, 45) configured to perform notification of the newly generated group of data, information of the certain user, and information relating to the determination result,
wherein if it is determined by the user determination unit (7) that the newly generated group of data does not relate to the certain user, notification of a message for confirming a handling policy of the certain user with respect to the determination result is performed by the information notification unit.

6. The user authentication system (1) according to claim 1, wherein
if it is determined by the user determination unit (7) that the newly generated group of data does not relate to the certain user, the new bodily information of aid newly generated group of data is subjected to processing for storage in the storage unit (8) and is not made available for other processing.

## Patentansprüche

1. Benutzerauthentifizierungssystem (1), umfassend:
mehrere Typen von Körperinformationen-Erfassungsvorrichtungen (10, 20, 30, 40), die eingerichtet sind, um unterschiedliche Typen von Körperinformationen für einen bestimmten Benutzer zu erfassen;
eine Steuereinheit (31, 41), die eingerichtet ist, um jedes Mal eine Gruppe von zugeordneten Daten bezüglich der Körperinformationen zu erzeugen, wenn neue Körperinformationen durch eine bestimmte Körperinformationen-
Erfassungsvorrichtung gemessen werden, wobei die Gruppe von Daten mindestens Informationen über Datum und Uhrzeit einer Messung, die Identifikationsnummer des Benutzers, die Informationen über die bestimmte Vorrichtungsidentifikationsnummer und die gemessenen Körperinformationen umfasst,
eine Speichereinheit (8), die eingerichtet ist, um die neu erzeugte Gruppe von Daten zu speichern;
eine Benutzerbestimmungseinheit (7), die eingerichtet ist, um zu überprüfen, ob es eine logische Diskrepanz zwischen der neu erzeugten Gruppe von Daten für den Benutzer und
einer weiteren zuvor gespeicherten Gruppe von Daten für denselben Benutzer gibt, wobei die zuvor gespeicherte Gruppe von Daten einer Körperinformationen-Erfassungsvorrichtung zugeordnet ist, die nicht die bestimmte Körperinformationen-Erfassungsvorrichtung ist, und um dadurch zu bestimmen, ob sich die neu erzeugte Gruppe von Daten auf den bestimmten Benutzer bezieht;
**dadurch gekennzeichnet, dass** die gemessenen Körperinformationen der zuvor gespeicherten Gruppe von Daten von einem anderen Typ als die Körperinformationen der neu erzeugten Gruppe von Daten sind und eine logische Diskrepanz auf die Bestimmung, dass die Erfassung der neu erzeugten Daten zu dem Datum und der Uhrzeit der neu erzeugten Gruppe von Daten aus einer zeitlichen Sicht unmöglich ist, zurückzuführen ist.

2. Benutzerauthentifizierungssystem (1) nach Anspruch 1, wobei eine Speichereinheit (8) in jeder der Körperinformationen-Erfassungsvorrichtungen (10, 20, 30, 40) vorgesehen ist und/oder unabhängig von den Körperinformationen-Erfassungsvorrichtungen (10, 20, 30, 40) vorgesehen ist.

3. Benutzerauthentifizierungssystem (1) nach Anspruch 1, wobei, wenn durch die Benutzerbestimmungseinheit (7) bestimmt wird, dass sich die neu erzeugte Gruppe von Daten nicht auf den bestimmten Benutzer bezieht, Merkmalinformationen, die angeben, dass sich die neu erzeugte Gruppe von Daten nicht auf den bestimmten Benutzer bezieht, an die Körperinformationen der neu erzeugten Gruppe von Daten angehängt werden.

4. Benutzerauthentifizierungssystem (1) nach Anspruch 1, ferner umfassend
eine Informationen-Benachrichtigungseinheit (15, 25, 35, 45), die eingerichtet ist, um eine Benachrichtigung über die neu erzeugte Gruppe von Daten, Informationen des bestimmten Benutzers und Informationen bezüglich des Bestimmungsergebnisses durchzuführen,
wobei, wenn durch die Benutzerbestimmungseinheit (7) bestimmt wird, dass sich die neu erzeugte Gruppe von Daten nicht auf den bestimmten Benutzer bezieht, eine Benachrichtigung einer Nachricht bezüglich des Bestimmungsergebnisses durch die Informationen-Benachrichtigungseinheit durchgeführt wird.

5. Benutzerauthentifizierungssystem (1) nach Anspruch 1, ferner umfassend
eine Informationen-Benachrichtigungseinheit (15, 25, 35, 45), die eingerichtet ist, um eine Benachrichtigung über die neu erzeugte Gruppe von Daten, Informationen des bestimmten Benutzers und Informationen bezüglich des Bestimmungsergebnisses durchzuführen,
wobei, wenn durch die Benutzerbestimmungseinheit (7) bestimmt wird, dass sich die neu erzeugte Gruppe von Daten nicht auf den bestimmten Benutzer bezieht, eine Benachrichtigung einer Nachricht zum Bestätigen einer Handhabungsrichtlinie des bestimmten Benutzers in Bezug auf das Bestimmungsergebnis durch die Informationen-Benachrichtigungseinheit durchgeführt wird.

6. Benutzerauthentifizierungssystem (1) nach Anspruch 1, wobei, wenn durch die Benutzerbestimmungseinheit (7) bestimmt wird, dass sich die neu erzeugte Gruppe von Daten nicht auf den bestimmten Benutzer bezieht, die neuen Körperinformationen der neu erzeugten Gruppe von Daten einem Verarbeiten zur Speicherung in der Speichereinheit (8) unterzogen und für ein weiteres Verarbeiten nicht zur Verfügung gestellt werden.

## Revendications

1. Système d'authentification d'utilisateur (1), comprenant :
une pluralité de types d'appareils d'acquisition d'informations physiques (10, 20, 30, 40) configurés pour acquérir différents types d'informations physiques pour un utilisateur;
une unité de commande (31, 41) configurée pour générer un groupe de données associées relatives aux informations physiques chaque fois que de nouvelles informations physiques sont mesurées par un certain appareil d'acquisition d'informations physiques, le groupe de données comprenant au moins des informations de la date et de l'heure d'une mesure, le numéro d'identification de l'utilisateur, les informations du numéro d'identification du certain appareil, et les informations physiques mesurées;
une unité de stockage (8) configurée pour stocker le groupe de données nouvellement généré;
une unité de détermination d'utilisateur (7) configurée pour vérifier si oui ou non il existe une différence logique entre le groupe de données nouvellement généré pour l'utilisateur et un autre groupe de données précédemment stocké pour le même utilisateur, ledit groupe de données précédemment stocké étant associé à un appareil d'acquisition d'informations physiques autre que ledit certain appareil d'acquisition d'informations physiques, et déterminer ainsi si oui ou non le groupe de données nouvellement généré se rapporte au certain utilisateur; **caractérisé en ce que** les informations physiques mesurées dudit groupe de données précédemment stocké sont d'un type différent de celui des informations physiques du groupe de données nouvellement généré et la différence logique est due à la détermination du fait que l'acquisition des données nouvellement générées à la date et à l'heure du groupe de données nouvellement généré est impossible dans une perspective temporelle.

2. Système d'authentification d'utilisateur (1) selon la revendication 1, dans lequel
une dite unité de stockage (8) est prévue dans chacun des appareils d'acquisition d'informations physiques (10, 20, 30, 40) et/ou est fournie indépendamment des appareils d'acquisition d'informations physiques (10, 20, 30, 40).

3. Système d'authentification d'utilisateur (1) selon la revendication 1, dans lequel
s'il est déterminé par l'unité de détermination d'utilisateur (7) que le groupe de données nouvellement généré ne porte pas sur le certain utilisateur, des informations d'attribut indiquant que les données ne portent pas sur le certain utilisateur sont jointes au groupe d'informations physiques nouvellement généré du groupe de données nouvellement généré.

4. Système d'authentification d'utilisateur (1) selon la revendication 1, comprenant en outre
une unité de notification d'informations (15, 25, 35, 45) configurée pour effectuer une notification du groupe de données nouvellement généré, d'informations du certain utilisateur, et d'informations relatives au résultat de la détermination,
dans lequel, s'il est déterminé par l'unité de détermination d'utilisateur (7) que le groupe de données nouvellement généré ne se rapporte pas au certain utilisateur, la notification d'un message se rapportant au résultat de la détermination est effectuée par l'unité de notification d'informations.

5. Système d'authentification d'utilisateur (1) selon la revendication 1, comprenant en outre
une unité de notification d'informations (15, 25, 35, 45) configurée pour effectuer une notification du groupe de données nouvellement généré, d'informations du certain utilisateur, et d'informations relatives au résultat de la détermination,
dans lequel, s'il est déterminé par l'unité de détermination d'utilisateur (7) que le groupe de données nouvellement généré ne se rapporte pas au certain utilisateur, la notification d'un message de confirmation d'une politique de traitement du certain utilisateur en ce qui concerne le résultat de la détermination est effectuée par l'unité de notification d'informations.

6. Système d'authentification d'utilisateur (1) selon la revendication 1, dans lequel
s'il est déterminé par l'unité de détermination d'utilisateur (7) que le groupe de données nouvellement généré ne se rapporte pas au certain utilisateur, les nouvelles informations physiques dudit groupe de données nouvellement généré sont soumises à un traitement pour le stockage dans l'unité de stockage (8) et ne sont pas rendues disponibles pour d'autres traitements.
